# EUROPÄISCHE PATENTSCHRIFT

(11) **EP 0 627 422 B1**
(45) Veröffentlichungstag und Bekanntmachung des Hinweises auf die Patenterteilung: **09.04.1997**
(21) Anmeldenummer: 94108389.1
(22) Anmeldetag: 31.05.1994
(51) Int. Cl.: C07D 213/81, C07D 215/48, C07D 215/50, C07D 241/24, C07B 43/06

(54) **Verfahren zur Herstellung von carboxamiden stickstoffhaltiger aromatischer Heterocyclen**
Process for the preparation of carboxamides of nitrogen-containing aromatic heterocycles
Procédé pour la préparation de carboxamides de composés hétérocycliques contenant de l'azote

(30) Priorität: 01.06.1993 CH 1630/93
(43) Veröffentlichungstag der Anmeldung: 07.12.1994
(60) Teilanmeldung: 96107208.9
(73) Patentinhaber: LONZA AG, CH-3945 Gampel/Wallis (CH)
(72) Erfinder: Roduit, Jean-Paul, Dr., Sierre (Kanton Wallis) (CH); Wellig, Alain, Ried-Mörel (Kanton Wallis) (CH); Amacker, Karin, Eischoll (Kanton Wallis) (CH); Eyer, Martin, Dr., Glis (Kanton Wallis) (CH)
(74) Vertreter: Weinhold, Peter, Dr.

(56) Entgegenhaltungen:
- DE-A- 2 056 433
- DE-B- 2 539 435
- US-A- 2 780 624

## Beschreibung

Die Erfindung betrifft ein neues Verfahren zur Herstellung von Carboxamiden stickstoffhaltiger aromatischer Heterocyclen (N-Heterocyclen).

Sowohl die Carboxamide als auch die aus diesen durch alkalische Hydrolyse hergestellten Carbonsäuren der N-Heterocyclen sind wichtige Zwischenprodukte zur Herstellung von Pharmazeutika.

Eine Carbamoylierungs-Methode von N-Heterocyclen ist aus der DE-PS 2 056 433 bekannt. Dieses Verfahren zeichnet sich dadurch aus, dass zur Bildung des Carbamoylradikals ein Redox-System aus R-OOH + Eisen (II) verwendet wird. R bedeutet hier Wasserstoff, Alkyl oder Cycloalkyl.
Eisen (II) muss dabei in stöchiometrischen Mengen oder sogar im Überschuss angewendet werden, was offensichtlich bei Synthesen im technischen Massstab grosse Abwasser- und Abfallprobleme mit sich bringt. Die verwendeten Alkylhydroperoxide sind teuer und zudem wegen ihrer Brisanz gefährlich zu handhaben.

Aufgrund dieser geschilderten Nachteile bestand die Aufgabe,ein Carbamoylierungsverfahren zu erarbeiten, dass im technischen Massstab einsetzbar ist und ohne diese Mängel funktioniert.

Mit dem Verfahren gemäss Patentanspruch 1 konnte die Aufgabe auf überraschend einfache Weise gelöst werden.

Unter N-Heterocyclen werden zweckmässig Verbindungen aus der Reihe der Pyridine, Chinoline, Isochinoline, Pyrimidine, Pyridazine, Pyrazine, Chinoxaline, Chinazoline, Acridine oder der Benzimidazole verstanden.
Diese Verbindungen können gegebenenfalls mit einem oder mehreren Substituenten aus der Reihe Alkyl, Alkoxy, Alkanoyl, Alkoxycarbonyl, Arylalkyl, Aryloxycarbonyl, Halogen, Carboxyl, Cyan, Amino, Alkylamino oder Dialkylamino versehen sein.

Die in den genannten Substituenten vorkommenden Alkylgruppen sind entweder linear oder verzweigt und haben zweckmässig 1 bis 6,bevorzugt 1 bis 4,C-Atome.

Unter einer Arylgruppe wird zweckmässig eine gegebenenfalls mit den genannten Substituenten substituierte Phenylgruppe verstanden.
Halogen steht üblicherweise für Fluor, Chlor, Brom oder Jod.

Formamid wird zweckmässig in einer Menge von 3 bis 35 mol, bevorzugt in einer Menge von 5 bis 6 mol,pro mol eingesetztem N-Heterocyclus, zugesetzt.
Peroxodischwefelsäure oder das Peroxodisulfat wird von Vorteil ebenfalls in einem leichten Überschuss,d.h. in einer Menge von zweckmässig 1.1 bis 3.0 mol pro mol eingesetztem N-Heterocyclus,zudosiert.
Von Vorteil werden die Peroxodisulfate der Peroxodischwefelsäure vorgezogen. Geeignete Peroxodisulfate sind das leicht zugängliche Natrium-, Kalium- oder Ammoniumperoxodisulfat.

Zur Verbesserung der Selektivität der Carbamoylierung wird in Gegenwart einer starken Säure, bevorzugt in Gegenwart von Schwefelsäure, gearbeitet.
Die Zugabe eines Lösungsmittels ist nicht zwingend, da grundsätzlich das im Überschuss angewendete Formamid diese Funktion übernehmen kann.
Allerdings ist es möglich, in Gegenwart eines polaren inerten Lösungsmittels zu arbeiten. Als besonders geeignet hat sich Acetonitril herausgestellt.

Die Reaktionstemperatur liegt zweckmässig zwischen 20° und 80°C, bevorzugt zwischen 65° und 75°C.

Das resultierende Carboxamid kann bereits nach relativ kurzer Zeit nach beendeter Zugabe von Peroxodischwefelsäure oder Peroxodisulfat auf fachmännische Weise aus dem Reaktionsgemisch isoliert werden. In der Regel fällt das Carboxamid in guter Ausbeute von grösser 80% und hoher Reinheit an.

Abhängig von der Substitution der eingesetzten N-Heterocyclen können bei der erfindungsgemässen Umsetzung zwei Carboxamidfunktionen eingeführt werden. Dies ist insbesondere bei "elektronenschiebenden" Gruppen wie z. B. bei den Alkylgruppen der Fall. So resultiert bei der erfindungsgemässen Umsetzung des 4-Methylpyridins das 4-Methylpyridin-2,6-dicarboxamid.

Die resultierenden Carboxamide können entweder isoliert werden oder direkt alkalisch zu den entsprechenden Carbonsäuren hydrolysiert werden. Enthalten die erfindungsgemäss hergestellten Carboxamide CN-Gruppen als Substituenten, werden diese in der Regel ebenfalls zur Carbonsäure hydrolysiert. So resultiert bei der alkalischen Hydrolyse des aus 4-Cyanpyridin hergestellten 4-Cyan-2-pyridincarboxamids die als Zwischenprodukt für Pharmazeutika bedeutsame Pyridin-2,4-dicarbonsäure.

### Beispiel 1

### Verfahren zur Herstellung von 4-Cyan-2-pyridin-carboxamid

85 g (0.82 mol) 4-Cyanpyridin wurden bei Raumtemperatur in 700 ml Acetonitril vorgelegt. Dann wurden 32.4 g (0.32 mol) Schwefelsäure 98% zugegeben. Die resultierende weisse Suspension wurde auf60°C erwärmt worauf 201.3 g (4.47 mol) Formamid in 52 g Wasser zugegeben wurde. Die erhaltene klare Lösung erhitzte man auf 70°C, worauf über einen Zeitraum von 2 Stunden 281.3 g (1.23 mol) Ammoniumperoxodisulfat portionenweise zudosiert wurde. (Exothermie!). Nach beendeter Zugabe wurde bei 74°C während 75 Minuten weitergerührt, dann 880 ml Wasser zugegeben und unter Vakuum das Azeotrop Wasser / Acetonitril abdestilliert. Die weiss-gelbe Suspension wurde dann bei 80°C filtriert, der Filterkuchen mit 80°C warmem Wasser gewaschen und unter Vakuum getrocknet.
Man erhielt 117 g (87.4%) des Titelproduktes mit einem Gehalt von ca. 90% (HPLC).

Die nachfolgenden Beispiele wurden analog zu Beispiel 1 durchgeführt

| Beispiel | Ausgangsprodukt | Produkt | Ausbeute |
|---|---|---|---|
| 2 | Pyridin-2,3-dicarbonsäure | 5-Carbamoyl-pyridin-2,3-dicarbonsäure | 70% |
| | | | |
| 3 | 4-Methylpyridin | 4-Methylpyridin-2,6-dicarboxamid | 81% |
| | | | |
| 4 | 4-Chlorpyridin | 4-Chlorpyridin-2-carboxamid | 55% |
| | | | |
| 5 | 2-Methyl-5-ethylpyridin | 6-Methyl-3-ethylpyridin-2-carboxamid | 40% |
| | | | |
| 6 | 2,5-Dimethylpyrazin | 3,6-Dimethyl-2,5-pyrazindicarboxamid | 15% |
| | | | |
| 7 | 2-Methylchinolin | 2-Methyl-4-chinolincarboxamid | 90% |
| | | | |
| 8 | 6-Methyl-2-pyridincarbonitril | 6-Cyan-2-methyl-3-pyridincarboxamid | 25% |
| | | | |
| 9 | 6-Chlor-2-pyridincarbonitril | 6-Cyan-2-chlor-3-pyridincarboxamid | 30% |

### Beispiel 10

### Verfahren zur Herstellung von 2-Carbamoyl-pyridin-4-carbonsäure aus Isonikotinsäure

80 g (0.65 mol) Isonikotinsäure wurden bei Raumtemperatur in 600 ml Acetonitril suspendiert. Dann wurden 26 g (0.26 mol) Schwefelsäure 98%, 161 g (3.58 mol) Formamid und 42.4 g Wasser zugegeben.
Die Suspension wurde auf 70°C erwärmt, worauf portionenweise 208.7 g (0.91 mol) Ammoniumperoxodisulfat so zugegeben wurde, daß die Temperatur 75°C nicht überstieg. Nach Rühren während 90 Minuten bei 73°C wurden 650 ml Wasser hinzugefügt. Darauf wurde die Suspension filtriert und der Filterkuchen mit 150 ml Wasser gewaschen. Es wurde 2-Carbamoyl-pyridin-4-carbonsäure erhalten.

## Patentansprüche

1. Verfahren zur Herstellung von Carboxamiden von stickstoffhaltigen aromatischen Heterocyclen, bei welchem eine stickstoffhaltige aromatische heterocyclische Verbindung mit Formamid in Gegenwart einer starken Säure umgesetzt wird, dadurch gekennzeichnet, daß zusätzlich in Gegenwart von Peroxodischwefelsäure oder einem Peroxodisulfat gearbeitet wird.

2. Verfahren nach Patentanspruch 1, dadurch gekennzeichnet, daß in Gegenwart von einem Peroxodisulfat gearbeitet wird.

3. Verfahren nach Patentanspruch 2, dadurch gekennzeichnet, daß Ammoniumperoxodisulfat eingesetzt wird.

4. Verfahren nach einem der Patentansprüche 1 bis 3, dadurch gekennzeichnet, daß die Peroxodischwefelsäure oder das Peroxodisulfat in einer Menge von 1.1 bis 3.0 mol pro mol eingesetzten N-Hetetocyclus eingesetzt wird.

5. Verfahren nach einem der Patentansprüche 1 bis 4, dadurch gekennzeichnet, daß das Formamid in einer Menge von 3 bis 35 mol pro mol eingesetzten N-Heterocyclus eingesetzt wird.

6. Verfahren nach einem der Patentansprüche 1 bis 5, dadurch gekennzeichnet, daß die starke Säure Schwefelsäure ist.

7. Verfahren nach einem der Patentansprüche 1 bis 6, dadurch gekennzeichnet, daß die Umsetzung bei einer Temperatur zwischen 20° and 80°C durchgeführt wird.

## Claims

1. Method for preparing carboxamides of nitrogen-containing aromatic heterocyclic compounds, wherein a nitrogen-containing aromatic heterocyclic compound is reacted with formamide in the presence of a strong acid, characterised in that the reaction is carried out in addition in the presence of peroxodisulphuric acid or of a peroxodisulphate.

2. Method according to claim 1, characterised in that the reaction is carried out in the presence of a peroxodisulphate.

3. Method according to claim 2, characterised in that ammonium peroxodisulphate is used.

4. Method according to one of claims 1 to 3, characterised in that the peroxodisulphuric acid or the peroxodisulphate is used in a quantity of from 1.1 to 3.0 mol per mol of N-heterocyclic compound used.

5. Method according to one of claims 1 to 4, characterised in that the formamide is used in a quantity of from 3 to 35 mol per mol of N-heterocyclic compound used.

6. Method according to one of claims 1 to 5, characterised in that the strong acid is sulphuric acid.

7. Method according to one of claims 1 to 6, characterised in that the reaction is carried out at a temperature of between 20°C and 80°C.

## Revendications

1. Procédé pour la préparation de carboxamides de composés hétérocycliques aromatiques contenant de l'azote, procédé dans lequel on met en réaction un composé hétérocyclique aromatique contenant de l'azote avec du formamide en présence d'un acide fort, caractérisé en ce que l'on travaille de plus en présence d'acide peroxosulfurique ou de peroxodisulfate.

2. Procédé selon la revendication 1, caractérisé en ce que l'on travaille en présence d'un peroxodisulfate.

3. Procédé selon la revendication 2, caractérisé en ce que l'on met en oeuvre du peroxodisulfate d'ammonium.

4. Procédé selon l'une des revendications 1 à 3, caractérisé en ce que l'on met en oeuvre l'acide peroxosulfurique ou le peroxodisulfate dans une quantité de 1,1 à 3,0 moles par mole de composé hétérocycle-N mis en oeuvre.

5. Procédé selon l'une des revendications 1 à 4, caractérisé en ce que l'on met en oeuvre le formamide dans une quantité de 3 à 35 moles par mole de composé N-hétérocyclique.

6. Procédé selon l'une des revendications 1 à 5, caractérisé en ce que l'acide fort est l'acide sulfurique.

7. Procédé selon l'une des revendications 1 à 6, caractérisé en ce que la réaction s'effectue à une température entre 20° et 80°C.
